Europäisches Patentamt

**European Patent Office**

Office européen des brevets

(11) Publication number: **0 238 851 B1**

# EUROPEAN PATENT SPECIFICATION

(45) Date of publication of patent specification: **09.12.92**     (51) Int. Cl.⁵: **G01N 33/50**, //G01N33/53, G01N33/68,C12Q1/18

(21) Application number: **87102387.5**

(22) Date of filing: **19.02.87**

(54) **A method and means of assaying an immune system.**

(30) Priority: **19.02.86 US 830728**
**24.02.86 US 832016**

(43) Date of publication of application:
**30.09.87 Bulletin 87/40**

(45) Publication of the grant of the patent:
**09.12.92 Bulletin 92/50**

(84) Designated Contracting States:
**AT BE CH DE FR GB IT LI NL SE**

(56) References cited:
**EP-A- 0 173 889**
**EP-A- 0 227 335**
**EP-A- 0 230 052**
**FR-A- 2 355 292**

**IMMUNOLOGICAL COMMUNICATIONS, vol. 10, no. 4 & 5, 1981, M. Dekker, Inc.; S.E.KELLER et al., pp. 417-431**

**PATENT ABSTRACTS OF JAPAN, vol. 6, no. 82 (P-116)[960], 20 May 1982**

(73) Proprietor: **Imreg, Inc.**
**Suite 1400, 144 Elk Place**
**New Orleans, LA 70112(US)**

(72) Inventor: **Gottlieb, A.Arthur, Dr. c/o Imreg, Inc.**
**Suite 1400**
**144 Elk Place**
**New Orleans, La. 70112(US)**

(74) Representative: **Popp, Eugen, Dr. et al**
**MEISSNER, BOLTE & PARTNER Widenmayer-strasse 48 Postfach 86 06 24**
**W-8000 München 86(DE)**

**Description**

**BACKGROUND OF THE INVENTION**

The present invention relates to means of assaying the immune function of a human being or animal. In a number of diseases or other pathological conditions, the immune system response of a human or animal subject is depressed. As a result the subject becomes more susceptible to opportunistic infections, malignancies, or other pathological conditions against which a normal immune system would have protected the subject. Among the pathological conditions that depress the immune system are Acquired Immune Deficiency Syndrome (AIDS) and AIDS-related complex (ARC). Chemotherapy and aging are also associated with immune deficiency. It is often desirable, in subjects suffering from such conditions, to assay the subject's immune function response to determine the extent of impairment.

In other conditions or circumstances, immune system response may be excessive relative to what is medically desired. When the immune system is not appropriately regulated, several systemic malfunctions can occur. For example, in certain autoimmune disorders the body reacts against its own tissue as if the tissue were a foreign body. Rheumatoid arthritis, multiple sclerosis, myasthenia gravis, lupus erythematosus, and insulin-dependent diabetes (type 1) are believed to be examples of such conditions. Also, when organ transplants are required, it is often medically desirable to lessen the subject's immune system response to avoid rejection of the organ transplant. In such circumstances, it can be desirable to assay the level of immune function response to determine how much greater it is than is desired.

Convenient, inexpensive, in vitro diagnostic tests for diagnosing impaired capacity or reserve of immune function are presently unavailable. In particular, quantitative tests are unavailable, although they would be very useful for several purposes in therapeutic regimes. Such tests would be helpful in assessing the remaining immune function of a person whose immune system has been damaged (for example, by chemotherapy or AIDS). The test would provide a diagnostic indicating the need for or feasibility of treatment and the appropriate extent thereof. Such tests would also be helpful in titrating to an acceptable amount the dosage of substances known to impair immune function, in titrating the dosage of substances deliberately administered to modify (suppress or amplify) immune function, and qualitatively in determining whether a person or animal suffers from a condition impairing its immune function.

An abstract published in March 1985 (Sizemore, Farmer, and Gottlieb, "Enhancement of nonspecific suppressor T cell activity by immunomodulators derived from human leukocyte dialysates," Fed. Proceedings 947, No. 3133) refers in general terms to various aspects of the procedures described herein. Other background on amplifiers and suppressors is found in Gottlieb U.S. Pat. Nos. 4,468,379 and 4,616,0379, and in corresponding Gottlieb EPO patent application No. EP-A-0 173 889, a document which belongs to the prior art by virtue of the provisions of Article 54(3) EPC.

The terms "amplifier" and "suppressor" are used hereinafter generally as in the cited Gottlieb patents and patent application. In very simple terms, "amplifiers" and "suppressors" are both "immunomodulators," i.e., materials that nonspecifically modulate the response of the immune system of an animal or human subject to antigens. Amplifiers increase, and suppressors decrease, such response. For a more precise explanation of the terms, the cited references should be consulted.

A known method of cultivating suppressor cells is to expose normal lymphocytes to a plant product known as Concanavalin A (Con A), which in certain concentrations induces the production of suppressor cells. By culturing lymphocytes with Con A, suppressor cells can be produced. See generally Shou, Schwartz, and Good, Suppresor cell activity after concanavalin A treatment of lymphocytes from normal donors, 143 J. Exp. Med. 1100 (1976); Birnbaum and Swick, Human suppressor lymphocytes, 40 Cell Immunol. 16 (1978). It is also known that proliferation of cells may be inhibited by administration of such agents as mitomycin, which interferes with cell division.

**SUMMARY OF THE PRESENT INVENTION**

The present invention concerns in vitro test methods for determining the magnitude of immune capacity or reserve function of an animal or human subject by assaying the ability of the subject's leukocytes to produce materials associated with the operation of the immune system. The procedure of this invention begins with isolation, by known procedures, of peripheral blood lymphocytes (PBLs, also referred to as peripheral blood mononuclear cells). The PBLs are exposed in vitro to mitogen or antigen.

One aspect of the invention then involves assaying the production of interleukin-2 (IL-2) in response to mitogen, in order to determine how impaired a test subject's immune system has become and to determine its potient of being restored to a higher level of activity. This is a measure of the positive (amplifier) reserve capacity of the subject. (The preferred embodiment uses the mitogen phytohemoagglutinin, PHA, but pokeweed mitogen, PWM, has also been used successfully; the procedure is not restricted to any particular mitogen. As indicated above, tetanus toxoid is usable; other antigens may be used that provoke an immune system response. Moreover, while the preferred embodiment is based on assaying the production of IL-2, other immune response products may be used for the assay, such as gamma-interferon and generation of cytotoxic lymphocytes, and the procedure is not restricted to any particular such index of immune response.)

In the case of a subject with an impaired immune system, the subject's mitogen-induced IL-2 production in vitro is substantially lessened, in the absence of addition of immunoamplifiers. However, the mitogen-induced production of IL-2 is increased substantially, in the case of such subjects having residual or remaining immune response, by the addition of amplifiers. On the other hand, when such subjects have very little or no immune reserve, amplifiers have no significant effect in increasing IL-2 production. The ability of amplifiers to increase mitogen-induced IL-2 production in vitro thus provides a convenient means to assay immune reserve, and thus assay the level to which the subject's immune function can be raised by treatment.

Another aspect of the invention involves assaying the ability of the subject's immune system to produce or potentiate (hereinafter referred to collectively as "to activate") suppressor cells. The subject's PBLs ("first PBLs") are exposed to a mitogen or other agent that stimulates activation of suppressor cells, and concurrently to several dilutions of amplifier and also a control. The procedure then involves removal of the remaining mitogen and treatment with an agent that prevents further proliferation of suppressor cells. A fresh PBL moiety ("second PBLs") is added and the mixture is exposed to another mitogen. The response of the second PBLs to this mitogen is decreased proportionally to the amount of suppressor activity induced in the first PBL moiety. Hence, the procedure permits a measurement to be made of the ability of the subject's PBLs to activate suppressors, in that the amount by which activation by the second PBLs is reduced is a measure of the suppressor capability of the first PBLs. This is a measure of the negative (suppressor) reserve capacity of the subject.

In measuring the reserve positive capacity of a subject's immune system to respond to antigen (i.e., reserve amplifier function), the preferred embodiment of the invention utilizes an assay that measures production of IL-2 or gamma interferon (G-IFN). In measuring the reserve negative capacity of a subject's immune system (i.e., reserve suppressor function), the preferred embodiment of the invention utilizes a proliferative response assay, namely, uptake of labelled thymidine into responder cells. It is considered inappropriate to use a proliferative response assay for reserve positive function, because, among other reasons, the amplifiers now available do not themselves stimulate cell proliferation; rather, they stimulate IL-2 and G-IFN production.

## DETAILED DESCRIPTION OF PREFERRED EMBODIMENTS

The procedures of this invention provide means of assaying immune function. The basic steps are preparation of purified peripheral blood mononuclear cell (PBL) populations which have been extracted from an animal or human subject, addition of mitogen to the purified PBL populations (with and without amplifier reagent), and assay of IL-2 production or, in the case of the auppressor assay, proliferation. All references below to Amplifier Beta, Zeta-2 and Eta are in terms of comparative examples only, and do not form part of the invention as claimed in the present application.

## I. Isolation and fractionation of peripheral lymphocytes

Venous blood (heparinized) was extracted from normal subjects and from patients with AIDS or ARC. An appropriate sample is approximately 20 to 25 ml/patient.

## Example 1 - Isolation of PBLs

Peripheral blood mononuclear cells (PBLs) were obtained from heparinized blood samples, by density gradient centrifugation on Lymphocyte Separation Medium (LSM) (Litton Bionetics, Kensington, Md., USA). The cells of interest are those found at the interface. The cells were washed 3x in RPMI 1640 medium containing 25 mM HEPES (GIBCO, Grand Island, NY). The cells were then resuspended to $10^6$ cells/ml in 25 mM HEPES, which may be supplemented with 10% normal human AB+ serum, 2mM L-glutamine (GIBCO), and 2% penicillin-streptomycin-neomycin antibiotic mixture (PSN antibiotic mixture, GIBCO).

## II. Amplifier Bioassay

Amplifiers used are TG (Tyrosylglycine or Tyr-Gly) and TGG (Tyrosylglycylglycine or Tyr-Gly-Gly), which are active peptide components of Beta, as described in EPO patent application EP-A-0 230052.

### A. Mitogen exposure and culture

Preparations of Amplifier Beta (the product concentration being that amount of amplifier derived from $400 \times 10^6$ buffy coat leukocytes, in each 1 ml of aqueous saline) were diluted with aqueous saline to provide the following reagents:

| Reagent | A | B | C |
|---|---|---|---|
| Dilution, 1: | 500 | 1000 | 2000. |

Reagent D was prepared, also, consisting of sterile normal saline.

The dilutions of Reagents A, B, and C correspond approximately to the amount of Amplifier Beta recovered by the inventor's process from 800,000, 400,000, and 200,000 leukocytes, respectively. The inventor has determined empirically that those three dilutions, in the procedure of Example 2, are satisfactory to accomplish the kind of assay described herein.

Those skilled in the art will appreciate, however, by examining the data described below, that other, as well as more or fewer, dilutions of this type may be selected to accomplish the kind of results one desires, using this disclosure as a guide. For example, more closely spaced dilutions -- such as 1:200, 300, 400, 500, 600, 800, 1000, 1200, 1500, 1800, 2000, 2400, 3000 -- will more precisely measure the maximum IL-2 production achievable. That procedure, however, will also (1) require extraction of more blood from the test subject and (2) be more laborious and costly. Clearly, tradeoff considerations must determine the number of dilutions made and their spacing. Accordingly, the inventor does not consider the invention to be limited to the particular quantities of reagent specified in the examples. Rather, the invention includes the use of such other proportions, as well, that the disclosure would teach a skilled person to utilize with necessary experimentation as suggested by the disclosure.

Furthermore, it may be appropriate to use much greater dilutions than those indicated -- in order to measure maximum IL-2 production obtainable. In some circumstances, much more dilute preparations of amplifier have yielded a maximum response, such as approximately $10^{-10}$ concentration.

### Example 2 - Preparation of mitogen-containing culture

PBLs of Example 1 are obtained from test subjects. The PBLs are resuspended to $1 \times 10^6$ cells/ml in RPMI 1640 medium containing 25 mM HEPES, supplemented with 10% FCS, 100 ug/ml streptomycin, and 100 U/ml penicillin. (The lower case letter **u** is used hereinafter to represent **micro**, as in **ug** or **ul**, for **micrograms** and **microliters**, respectively; the upper case letter **U** hereinafter represents **Units**.)

At least four 200 ul aliquots (and preferably a multiple of four, such as five sets of four, or 20, to permit subsequent pooling of aliquots for averaging purposes) of each test subject's resuspension are dispensed into the wells of 96-well microtiter plates (Falcon Labs #3072). To each of the four sets of each test subject's aliquots was added, respectively, 20 ul of Reagent A, B, C, or D. In addition, a suitable amount of PHA mitogen (Wellcome Reagents HA 16) is added to each well, in a volume of 20 ul/well.

The resulting materials are incubated at 37°C, 5% $CO_2$, for 24 hours. Replicate supernatants are harvested (pooled as appropriate), and frozen until assayed.

### B. IL-2 assay

IL-2 level was determined by measuring the incorporation of radioactively tagged thydiminine, 3H-TdR (New England Nuclear, Boston, Mass., USA), into the IL-2 dependent cell line CTLL-2 (American Type Culture Collection, Rockville, Md., USA), using a novel method that the inventor developed for this purpose, and departing in various respects, from that of Gillis et al., "T cell growth factor: parameters of production and a quantitative microassay for activity," 120 J. Immunology 2027 (1978).

### Example 3 - Amplifier assay procedure

Log phase CTLL-2 cells are resuspended to $10^5$/ml in an enriched assay medium (RPMI 1640, 25 mM HEPES supplemented with 15%-FCS, 50 uM 2-mercaptoethanol, 2 mM L-glutamine, 100 U/ml penicillin, 100 $\mu$g/ml streptomycin). 100 $\mu$l aliquots are distributed to the wells of 96-well flat bottom microtiter plates.

The frozen supernatants of Example 2 are thawed. Serial dilutions are prepared, with aqueous saline, to provide undiluted, 50%, and 25% samples, respectively, which are distributed to the wells for culturing.

The cultures are incubated at 37° C, 5% $CO_2$, for 24 hours; 1 uCi/well 3H-TdR (New England Nuclear, Boston, Mass.) is added for the terminal 5 hours of incubation. The cultures are harvested on filter discs using a MASH II unit by standard procedures, including washing, which are well known in the art.

The filter discs (containing the cells) are subjected to liquid scintillation counting to determine the amount of 3H thymidine incorporated. Counts per minute (**cpm**) are recorded and the data is analyzed as described below. In the absence of IL-2, CTTL-2 cells in this system incorporate thymidine in an amount causing a reading of less than 500 cpm/culture. Maximal incorporation in the presence of IL-2 runs to approximately 30,000 to 40,000 cpm/culture.

An alternative assay method may be used in which thymidine is tagged with another measuring means, such as a fluorescent dye or fluorescent antibody.

### C. Reference preparation

A standard IL-2 preparation was made for relative activity comparison, using 3H-Tdr. It was prepared by incubating $10^6$/ml rat splenocytes with 10 ug/ml Con A (Sigma Chem. Co., St. Louis, Mo., USA, "Concanavalin A," No. C-2010, Type IV) for 24 hours. This preparation was defined as 10 U/ml IL-2. Therefore, the following formula was established for U/ml IL-2:

$$\text{U/ml IL-2} = \frac{10 \times \text{dilution of experimental sample for 50\% maximum cpm}}{\text{dilution of rat standard for 50\% maximum cpm}}$$

Under these assay conditions, 50% of maximal 3H-TdR incorporation by CTTL-2 cells represents 0.7 U/ml of the reference human IL-2 preparation supplied by the U.S. National Cancer Institute's Biological Response Modifiers Program.

Normal subjects' PBLs generally yield from 0.9 to 7.0 U/ml IL-2 using 1 ug/ml PHA in the cultures, which is approximately an optimal PHA dosage. (Note: 1 ug/ml PHA is equivalent to 20 ul/well, using the procedure of Example 2.) Most such subjects fall between 1 and 3 U/ml.

### III. Results of amplifier assays

A description follows of representative results of the procedure described in Examples 2 and 3, when PHA was used as a mitogen to stimulate IL-2 production: Increased levels of IL-2 resulted when Amplifier Beta was added to PHA. Use of Amplifier Beta alone, however, did not result in IL-2 production in the absence of PHA. It was also observed that high concentrations of amplifier may cause a paradoxical or suppressive effect, which vanishes when the amplifier is diluted.

The data showed varying ratios of IL-2 production in response to PHA alone (Reagent D) and in response to PHA plus Amplifier Beta. For different subjects the concentration of PHA plus amplifier that produced maximum IL-2 production relative to IL-2 production under stimulation with PHA alone was different. The ratio between the highest IL-2 production for a Beta + PHA mixture (Reagents A, B, or C) and production for PHA without Beta (Reagent D) was calculated. (Ratios based on a zero or near-zero Reagent D production as denominator are disregarded, since such a ratio is essentially meaningless.) Both this ratio and the highest production of IL-2 (in U/ml) may be used as assay indicators.

In the case of three normal test subjects, the highest IL-2 production tested between 2.4 and 16.8 U/ml, and the ratio of highest production to control (Reagent D) production tested between 1.1 and 2.0. In the case of three ARC and two AIDS test subjects, the highest IL-2 production tested between 0.2 and 3.8 U/ml, and the ratio of highest production to control (Reagent D) production tested between 1.8 and 4.9.

Thus, in all cases, Amplifier Beta increased the in vitro measurement of immune response significantly. In the case of PBLs from normal persons, the IL-2 production stimulated by PHA + Beta was almost always a maximum of twice or more the production that PHA alone stimulated. In PBLs of ARC/AIDS patients, the same ratio ranged from somewhat below a factor of 2 in some cases to much greater improvements in half or more of the sample. In PBLs of most normal persons, the maximum IL-2 production that could be elicited was greater than the maximum production that could be elicited for PBLs of ARC/AIDS patients. In general, then, it may be concluded that Amplifier Beta causes a relatively greater percentage of increase on ARC/AIDS patient PBLs' IL-2 production than it does on normal person PBLs' production, but it usually cannot bring AIDS/ARC patient PBLs' amount of IL-2 production up to the same levels as normal person PBLs' maximum amount of production.

**IV. Use of TGG in Assaying Amplifier Function**

Preparations of TGG were prepared, to repeat the procedure described above. TGG, purified in accordance with the procedures of the cited co-pending EPO patent application, was prepared to various dilutions. The starting dilution was 1:1000, which is approximately $3 \times 10^{-10}$ M. A preparation of 1 $\mu$g/ml PHA was also prepared and used as before.

Example 4 - TGG tests

The procedures of Examples 2 and 3 are repeated with two normal test subjects, using TGG preparations instead of Preparations Amplifier Beta preparations.

In subject #1, IL-2 production tested 0.69 U/ml for control, and from 0.93 to 1.76 U/ml for the various dilutions of TGG. The maximum production was at 1:8000 dilution, which provided a 2.55 ratio over control.

In subject #2, IL-2 production tested 0.9 U/ml for control, and from 0.99 to 1.38 U/ml for the various dilutions of TGG. The maximum production was at 1 : 32,000 dilution, which provided a 1.53 ratio over control.

The data is generally consistent with that for Amplifier Beta, of which TGG is a component. The data indicates a normal PBL range of IL-2 production that can be approximately doubled by TGG, in vitro.

**V. Therapeutic utilization of "Immune Reserve" test**

It is clear from the preceding data that Amplifier Beta and TGG have the ability to enhance the ability of lymphocytes from immunodeficient patients to produce IL-2 in response to a suitable stimulus, such as a mitogen. The increment in IL-2 production is a measure of the potential capacity of such cells to produce IL-2. Because the patient's cells do not produce so great a quantity of IL-2 without Beta or TGG, even when the cells are given maximal mitogen stimulation, the increment in IL-2 production is a measure of the reserve (but not previously utilized) capacity of the test subject's cells to produce IL-2, and by inference to produce other immunoregulatory materials.

The experimental data discussed above leads to the provision of diagnostic methods, and compositions for performing such methods. As indicated earlier, the invention disclosed herein provides in vitro means of assaying human and animal immune response in a number of circumstances where such assays are useful for medical and veterinary purposes.

The following examples are intended to illustrate the use of the assay method described above. In these examples, the following terminology is at times used: "immune reserve" refers to the maximal immune response caused by mitogen plus amplifier (such as by use of Reagents A, B, or C, whichever produces a maximal response); "base immune response" refers to the immune response caused by mitogen alone (such as by use of Reagent D).

Example 5 - Determination of immune reserve

PBLs from a patient with immune system deficiency due to AIDS or ARC are assayed in accordance with the foregoing procedure using Amplifier Beta. The attending physician determines that the patient's immune reserve is sufficient to make it advisable, in the physician's medical judgment, to attempt to restore it by administration of an amplifier. (For example, his immune reserve is greater than 0.5 U/ml.)

Example 5A - Immune reserve of second patient

PBLs from a second patient with immune system deficiency due to AIDS or ARC are assayed in accordance with the same procedure. The attending physician determines that the patient's immune reserve is insufficient to make it advisable, in the physician's medical judgment, to attempt to restore it by administration of an amplifier. (For example, his immune reserve is below 0.1 U/ml.) No amplifier treatment is attempted.

It will be appreciated that the use of 0.1 U/ml as a predetermined reference level may be affected by other circumstances, such as availability of personnel, supplies, or facilties, and other conditions sometimes referred to under the rubric of "triage."

Example 5B - TGG immune reserve determination

Examples 5 and 5A are repeated using a TGG assay procedure instead. The results are the same.

Example 5C - TG immune reserve determination

Examples 5 and 5A are repeated using a TG assay procedure instead. The results are the same.

Example 5D - Zeta-2 immune reserve determination

Examples 5 and 5A are repeated using an assay procedure with Amplifier Zeta-2 of the copending EPO patent application, instead. The results are the same.

Example 5D - Eta immune reserve determination

Examples 5 and 5A are repeated using an assay procedure with Amplifier Eta of the copending EPO patent application, instead. The results are the same.

Example 6 - Titration of amplifier dosage

The physician of Example 5 administers to the patient of Example 5 a daily dosage of Amplifier Beta equivalent to that derived from 400,000 leukocytes. After one week the physician reassays the patient's immune response in accordance with the foregoing procedure. The physician determines, in his or her medical judgment, that the base immune response has now been restored to an acceptable level. (For example, base immune response of at least 1.0 U/ml.)

Thereafter, the physician administers to the patient a weekly dosage of Amplifier Beta equivalent to that derived from 400,000 leukocytes.

At monthly intervals, the physician reassays the patient's base immune response and the patient's immune reserve, and makes a determination whether the dose of Amplifier Beta should be increased or decreased, in order to maintain the base immune response at an acceptable level.

Example 6A - TGG titration of amplifier dosage

The procedure of Example 6 is repeated with the assay procedure using TGG instead. The results are the same.

Example 6B - TG titration of amplifier dosage

The procedure of Example 6 is repeated with the assay procedure using TG instead. The results are the same.

Example 6C - Zeta-2 titration of amplifier dosage

The procedure of Example 6 is repeated with the assay procedure using Amplifier Zeta-2 instead. The results are the same.

Example 6D - Eta titration of amplifier dosage

The procedure of Example 6 is repeated with the assay procedure using Amplifier Eta instead. The results are the same.

Example 7 - Titration of chemotherapy

PBLs from a patient undergoing chemotherapy are assayed in accordance with the foregoing procedure for Amplifier Beta. The attending physician determines that the patient's immune reserve has been lowered to a value below that which the physician, in his or her medical judgment, considers acceptable. (For example, below 0.5 U/ml.)

The physician decreases the chemotherapy dosage to 50% of its former level, or in the alternative prescribes twice-weekly dosages of Amplifier Beta (for example, each dose equivalent to material from 400,000 leukocytes). After one week the physician reassays the patient's immune reserve in accordance with the same procedure.

The physician determines, in his or her medical judgment, that the immune reserve is now at an acceptable level. (For example, approximately 0.8 U/ml.)

At weekly intervals, the physician reassays the patient's immune response and makes a determination whether the chemotherapy dosage is at as high a level as possible without lowering the patient's maximal immune response below a level that the physician considers acceptable. The procedure of the second paragraph of this Example is repeated or modified if necessary.

### Example 7A - TGG titration of chemotherapy

The procedure of Example 6 is repeated with the TGG assay procedure instead. The results are the same.

### Example 7B - TG titration of chemotherapy

The procedure of Example 6 is repeated with the TG assay procedure instead. The results are the same.

### Example 7C - Zeta-2 titration of chemotherapy

The procedure of Example 6 is repeated with Amplifier Zeta-2 instead. The results are the same.

### Example 7D - Eta titration of chemotherapy

The procedure of Example 6 is repeated with Amplifier Eta instead. The results are the same.

The foregoing assay procedure may be expanded to use in cows, pigs, and other animals, by extrapolation from the data on human beings, in a manner that will be obvious to those skilled in the art.

## VI. Gamma-interferon Assay of "Immune Reserve"

The general IL-2 assay method described above can be modified to include any measurable lymphokine (biological response modifier). For example, gamma-interferon can also be measured on the same supernatants used for IL-2. Quantitation of gamma-interferon in such culture supernatants is advantageously accomplished by using a solid phase radioimmunoassay marketed by Centocor Inc., Malvern, Pa., which employs two monoclonal antibodies specific for gamma-interferon. Example 8 illustrates this assay using PBLs from an AIDS patient. Preparations. E and F of 0.5 and 1.0 ug/ml PHA, respectively, are prepared. Preparations G, H, I, and J of sterile saline, and 1:500, 1:1000, and 1:2000 Amplifier Beta, respectively, are prepared. This permits eight tests, using Preparations E and F respectively against Preparations G, H, I, and J. A reference is established, as before.

### Example 8 - Gamma-interferon assay

PBLs from an AIDS patient are prepared and the procedures of Examples 2 and 3 are repeated, with monoclonal antibody assay for gamma-interferon substituted for thymidine uptake assay for IL-2. Preparations E through J are used, the results are observed, and the results are tabulated in Table 5.

For PHA concentration 0.5 ug/ml control gamma-interferon production is 20 U/ml. Dilutions 1:500, 1:1000, and 1:2000 result in 28; 30, and 32 U/ml respectively. For PHA concentration 1.0 ug/ml, the corresponding results are 52, 74, 68, and 82 U/ml. Both maximum ratios are approximately 1.6.

It is observed that gamma-interferon production is increased by Amplifier Beta. The preceding assays are radioimmunoassays. The procedure may, if desired, be modified to ELISA assays, which involve no radioisotope and instead provide a fluorescent readout. ELISA assays are well known in the art and require no detailed description here. See, e.g., E. Leinikki et al., ELISA Assay of Specific Rubella Antibody Levels, 8

8

J. Clin. Microbiol. 419 (1979); E. Engvall and P. Perlmann, ELISA: Quantitative Assay of Immunoglobulin, 8 Immunochem. 871 (1971). It should be noted that an ELISA assay may be used to assay IL-2 production, also, as well as that of other lymphokines. Furthermore, TGG, TG, Zeta-2, and Eta may be used instead of Beta in Example 8.

## VII. Activation and culture of suppressor cells for "Suppressor Reserve" Assay

### A. Activation with Con A

The PBLs preparation of Example 1 is activated with Con A or another agent that activates suppressor cells. The preparation is divided into parts and concurrently exposed to various dilutions of amplifier. Suitable amplifiers include Amplifier Beta, Amplifier Zeta-2, Amplifier Eta, TG, and TGG.

Example 9 - Con A activation of cells

Con A (Concanavalin A, Sigma Chem. Co., St. Louis, No. C-2010, Type IV) is prepared as a 1 mg/ml stock in saline, and the concentration is verified spectrophotometrically ($E = 1.14$ at 280 nm, 1 cm path length).

A sample of the cell preparation of Example 1 is mixed with a volume of a dilution of Con A stock that reduces Con A concentration to 0.2 to 0.5 ug/ml in the final preparation. The Con A-treated cells are set aside for the procedure of the next Example.

### B. Addition of Beta, Cultivation

Preparations of Amplifier Beta (the product concentration being that amount of amplifier derived from $400 \times 10^6$ buffy coat leukocytes, in each 1 ml of aqueous saline) were diluted with aqueous saline to provide the following reagents:

| Reagent | A | B | C | D | E | F |
|---|---|---|---|---|---|---|
| Dilution, 1: | 500 | 1000 | 2000 | $10^4$ | $10^5$ | $10^6$. |

Reagent G was prepared, also, consisting of sterile normal saline.

The dilutions of Reagents A, B, C, D, E, and F correspond approximately to the amount of Amplifier Beta recovered by the inventor's process from 800,000, 400,000, 200,000, 40,000, 4000, and 400 leukocytes, respectively. The inventor has determined empirically that those dilutions described below in examples, are satisfactory to accomplish the kind of assay described hereinafter. (Reagents A, B, and C were used for one test subject and Reagents D, E, and F were used for a second.)

As stated before, those skilled in the art will appreciate, however, by examining the data described below, that other, as well as more or fewer, dilutions of this type may be selected to accomplish the kind of results one desires, using this disclosure as a guide.

Example 9A - Adding Amplifier Beta and culturing cells (Subject #1)

To 20 ml of the Con-A treated cell preparations of Example 2A is added 20 ul of Preparation A. Four 5-ml samples of the resulting material are placed in 60 mm culture dishes (Corning, Corning, NY).

The same procedure is repeated successively with Preparations B, C, and G (control).

The dishes of cells are incubated for 48 hr at 37°C in a 5% $CO_2$ in air atmosphere. Cells are harvested and washed 3x with 0.2M alpha methyl mannoside (Sigma Chem. Co., Grade II, M-3752, St. Louis) in HBBS (Hanks Balanced Salt Solution, GIBCO) to remove residual Con A. (A, B, C, and G preparations are kept separate from one another.)

Example 10 - Exposure to and removal of mitomycin

Cells are resuspended to approximately $10^7$/ml in HBSS and are treated with mitomycin C (Sigma Chem., M-0503) at a final concentration of 50 ug/ml for 45-60 min at 37°C, in an atmosphere of 5% $CO_2$. This is followed with 3x washes with cold HBSS with 2% AB+ serum.

The cells are resuspended in RPMI-1640 medium containing 25 mM HEPES supplemented with 10% AB + serum and 2% PSN, and they are incubated for 1 hr at 37°C to allow for the release of any residual mitomycin C in culture. The cells are then washed 2x in RPMI-1640 and resuspended to 1.5 x $10^6$ cells per ml in RPMI-1640 medium containing 25 mM HEPES supplemented with 10% AB + serum and 2% PSN. The suspension is set aside. (As before, A, B, C, and G preparations are kept separate from one another.)

## VIII. Bioassay for Suppressor Immune Reserve

The non-proliferating suppressor culture of Example 10 is then mixed with a fresh PBL. Preferably, the test subject's PBLs should be used to avoid introducing another possible variable. But this is not always feasible, and therefore a PBL from a normal donor may be used. The new PBL is mixed with the culture of Example 10, exposed to a material (such as PHA) that will cause a cell proliferative response, and the proliferative response, as an index of immune function, is assayed.

Example 11 - Preparation of cultures for assay of suppressor reserve

Fresh PBLs of Example 1 are resuspended to 1.5 x $10^6$ cells/ml in RPMI-1640 medium containing 25 mM HEPES supplemented with 10% AB + serum and 2% PSN, thereby providing a Responder Preparation. The responder cells are dispensed in 0.1 ml volumes into wells of a 96-well microtiter plate (Falcon # 3072, Becton Dickinson, Oxnard, CA).

The suspensions of Example 10 are dispensed in 0.1 ml volumes into the wells containing the responder cells. It is desirable to do the procedure in triplicate or quadruplicate in order to average out error, so that 12-16 wells will be used.

A suitable concentration of mitogen, such as PHA (Wellcome Reagents HA16) or Pokeweed Mitogen (GIBCO, Grand Island, NY) or Con A is added to each well, in a volume of approximately 20 ul/well. A suitable concentration in the well of PHA for this purpose is 0.25 ug/ml. A suitable concentration in the well of Con A for this purpose is 1.0 ug/ml.

The plates are then incubated for 72 hr at 37°C in a 5% $CO_2$ in air atmosphere. At 18 hours before conclusion, 20 ul/well of 20 uC/ml tritiated thymidine (New England Nuclear, Boston MA) is added to each well.

The wells are subsequently harvested onto glass fibre filter strips (M.A. Bioproducts, Walkersville MD) and the resultant counts per minute (cpm) are determined by liquid scintillation counting.

Uptake of radioactive thymidine was used as a convenient measure of cell proliferation ("cell proliferation index") because it is relatively inexpensive and readily available. Uptake of other DNA precursors, such as deoxycytidine, could be used as a cell proliferation index, but they are believed to be not as convenient.

The following quantities are defined for the purposes of the assay of Example 11:

Corrected cpm = Mean cpm with mitogen - mean cpm without mitogen
% suppression = 100[1 - (corrected cpm with Con A-activated cells exposed to amplifier)/(corrected cpm with Con A-activated cells not exposed to amplifier)]

The % suppression results for Test Subject No. 1, for various dilutions of Amplifier Beta are as shown below:

Table 1

| Modulation of Suppressor Activity by Various Dilutions of Amplifier Beta (Subject #1) | | | | |
|---|---|---|---|---|
| Reagent | A | B | C | G |
| Dilution, 1:<br>% Suppression | 500<br>66% | 1000<br>42% | 2000<br>77% | (control)<br>0% |

The foregoing procedure was carried out with a second test subject, using Reagents D, E, F, and Reagent G (control).

Example 12 - Assay with Beta (Subject #2)

The procedure of Examples 9 to 11 was repeated with a second test subject, but in Example 9A Reagents D, E, and F were used in place of Reagents A, B, and C.

The results of the assay were that maximum % suppression was 57% at $10^{-4}$ dilution.

### IX. Use of Other Assay Amplifiers

The foregoing procedure may be carried out with other amplifiers, as well as with Amplifier Beta. Dilutions of Amplifier Zeta-2 were prepared from amplifier derived from $400 \times 10^6$ leukocytes per ml aqueous saline. That preparation was further diluted with aqueous saline to provide the following additional reagents:

| Reagent | H | I | J | K | L |
|---|---|---|---|---|---|
| Dilution, 1: | 1000 | 5000 | $10^4$ | $10^5$ | $10^6$. |

As before, Reagent G was sterile saline.

### Example 13 - Assay with Zeta-2 (Subject #3)

The procedure was repeated with a second test subject, but in Example 9A Reagents H, I, and J were used in place of Reagents A, B, and C.

The results of the assay were that maximum % suppression was 29.5 at $10^{-4}$ dilution.

### Example 14 - Assay with Zeta-2 (Subject #4)

The procedure was repeated with a second test subject, but in Example 9A Reagents J, K, and L were used in place of Reagents A, B, and C, and 1.0 ug/ml Con A was used to induce cell proliferation in the responder culture. Cultures were stimulated with 0.25 ug/ml PHA.

The results of the assay were that maximum % suppression was 47.1% at $10^{-5}$ dilution.

Preparations of TGG are prepared, to repeat the procedure described above. TGG is prepared to various dilutions. The starting dilution is 1:500, which is approximately $3 \times 10^{-10}$ M. Reagents M, N, and O are prepared, with further TGG dilutions of 1 to 1000, 2000, and 4000, respectively. As before the control is Reagent G.

### Example 15 - Assay with TGG

The foregoing procedure is repeated, but in the procedure of Example 9A Reagents M, N, and O are used in place of Reagents A, B, and C. The results of the assay are comparable to those of Examples 9 to 14.

As previously indicated, with reference to assay of cell proliferative response, an alternative assay method may be used in which thymidine is tagged with another measuring means, such as a fluorescent dye or fluorescent antibody. Moreover, ELISA assays may be used instead. As indicated above, an IL-2 assay is also a suitable index; use of a gamma-interferon assay is also appropriate.

### X. Therapeutic utilization of Suppressor Reserve test

It is clear from the preceding data that amplifiers have the ability to modify the suppressor response of lymphocytes. The inventor theorizes that amplifiers operate to suppress immune response through activation of a subpopulation of T4 cells known as suppressor-inducer cells. The inventor further theorizes that the immune response to mitogen caused by administration of amplifier is a mixture of positive ("amplifier") and negative ("suppressor") effects, with the latter preponderating in a dosage zone that may be referred to as the "paradoxical response" range. The inventor has observed, and the previously patents and patent applications note, that, when a range of concentrations of amplifier is applied to a test subject's PBLs, the magnitude of immune response decreases after a certain concentration is reached. That is, the immune response increases with increasing concentration of amplifier through a range that may be designated the "nonparadoxical response" concentration range, but after a maximum response is reached, further increasing concentrations of amplifier merely decrease the immune response; this is the "paradoxical response" concentration range.

In any event, the observed decrement in proliferative response to mitogen is believed to be a reflection of the potential ability of the patient's suppressor cells to be activated, and therefore a range of concentrations provides a measure of the maximum potential capacity of such cells to be activated. That is to say, the decrement in proliferative response is a measure of the reserve (but previously unutilized) capacity of the PBLs to produce suppressor cells and/or manifest suppressor function, and by inference to produce other immunoregulatory materials having a suppressive effect on the immune system.

As indicated earlier, the invention disclosed herein provides an in vitro means of assaying human and animal immune response in a number of circumstances where such assays are useful for medical and veterinary purposes. The following examples are intended to illustrate the use of the assay method described above. In these examples, the following terminology, which was used above, is at times used: "suppressor immune reserve" refers to the maximal suppressor response caused by the procedures described above. "Base suppressor response" refers to the suppressor response caused by mitogen Con A alone (such as by use of Reagent G in the Examples).

Example 16 - Determination of suppressor immune reserve

PBLs from a patient with immune system dysfunction associated with rheumatoid arthritis are assayed in accordance with Examples 9 to 14. The attending physician determines that the patient's suppressor immune reserve is sufficient to make it advisable, in the physician's medical judgment, to attempt to restore it by administration of an amplifier. (For example, suppressor immune reserve above 40 %.)

Example 17 - Suppressor immune reserve of second patient

PBLs from a second patient with immune system dysfunction associated with rheumatoid arthritis are assayed in accordance with the same procedure. The attending physician determines that the patient's suppressor immune reserve is insufficient to make it advisable, in the physician's medical judgment, to attempt to restore it by administration of an amplifier. (For example, suppressor immune reserve below 10%.) No amplifier treatment is attempted.

It will be appreciated that the use of 10% as a predetermined reference level may be affected by other circumstances, such as availability of personnel, supplies, or facilties, and other conditions sometimes referred to under the rubric of "triage."

Example 18 - Titration of amplifier dosage

The physician of Example 16 administers to the patient of Example 16 a daily dosage of Amplifier Beta equivalent to that derived from 40 million leukocytes. After one week the physician reassays the patient's base suppressor response in accordance with the foregoing procedure. The physician determines, in his or her medical judgment, that the base suppressor response has now been restored to an acceptable level. (For example, base suppressor response of at least 50% of tested suppressor immune reserve.)

Thereafter, the physician administers to the patient a weekly dosage of Amplifier Beta equivalent to that derived from 40 million leukocytes.

At monthly intervals, the physician reassays the patient's base suppressor response and makes a determination whether the dose of Amplifier Beta should be increased or decreased, in order to maintain the base suppressor response at an acceptable level.

Example 19 - Extreme reaction to poison ivy

A patient displays a hypernormal reaction to poison ivy or another hypernormal cell-mediated immune reaction to foreign antigen. The attending physician suspects a systemic dysfunction of the patient's suppressor function.

The physician performs an assay in accordance with the procedure of Example 16 and confirms the suspicion. The patient's suppressor immune reserve is not abnormally low, but the patient's base suppressor response is significantly below normal. (For example, suppressor immune reserve is at least approximately 40%; base suppressor response is less than 20%.)

The physician administers Amplifier Beta or Amplifier Zeta-2 in the paradoxical response concentration range. For example, the physician administers to the patient every two days a dosage of Amplifier Beta equivalent to that derived from 40 million leukocytes. Treatment continues until base suppressor response is restored to at least approximately 45%.

The foregoing assay procedure may be expanded to use in cows, pigs, and other animals, by

EP 0 238 851 B1

extrapolation from the data on human beings, in a manner that will be obvious to those skilled in the art.

## GENERAL CONCLUDING REMARKS

As used in the claims, the term **peripheral blood lymphocyte** refers to the mononuclear cell populations (PBLs) isolated as described in Example 1, supra. However, the procedure of Example 1 may be varied in ways familiar to those skilled in the art, and the result will still be regarded by those skilled in the art as PBLs.

The term amplifier includes TG, TGG and their pharmaceutically acceptable salts, amides, esters, and protected derivatives thereof

The term **mitogen** ordinarily means any substance capable of inducing proliferation of lymphocytes, i.e., any means for inducing a cell proliferation response. Mitogens include, among other things, PHA, pokeweed, and tetanus toxoid. The term **antigen** means a defined substance, such as tetanus toxoid, that produces a specific immune response. As used in the claims, **mitogen** includes **antigen**, as well as **mitogen** as defined above. Further, mitogens and antigens are examples of means for activating an immune system response.

The term **cell proliferation index** means a measure of cell proliferation. Such a measure can be a direct measurement of cell proliferation, as for example, by measuring cell uptake of a material, such as thymidine, associated with cell proliferation. Those skilled in the art will recognize that other DNA precursors may be utilized in lieu of thymidine, such as deoxycytidine, deoxyadenosine, or other DNA precursors. (Those skilled in this art will recognize that such DNA precursors are equivalents of thymidine in the claims.)

A cell proliferation index is an example of an **index of immune system response**. Another example is gamma-interferon production. Another example is generation of cytotoxic lymphocytes.

The term **immunodeficient condition** as used in the claims includes systemic immune deficiency arising from, among other things, AIDS, ARC, chemotherapy, aging, cancer, and refractory infections. Moreover, certain apparent autoimmune conditions have immunodeficient components, as well, rheumatoid arthritis being an example.

The term **autoimmune condition** includes the collagen diseases, which those skilled in the art understand to have significant autoimmune components. As indicated earlier, rheumatoid arthritis, multiple sclerosis, myasthenia gravis, lupus erythematosus, and insulin-dependent diabetes (type 1) are believed to be examples of autoimmune conditions.

The effect of amplifier on immune system response can be represented as an increasing function of dosage amount within a certain range. At a certain point, however, increased dosages of amplifier decrease, rather than increase, the amount of immune response. This has been referred to earlier as the "paradoxical" effect. The first part of the dosage range of amplifier (beginning at zero amplifier dosage and continuing until the paradoxical effect sets in) may therefore be termed the **nonparadoxical dosage range**. The second part (beginning where the paradoxical effect sets in) may be termed the paradoxical dosage range.

The term tagged means labelled, such as radioactively or with a fluorescent dye.

The term **activation**, as applied to suppressor cells, includes stimulation of suppressor cell formation and potentiation of suppressor cell activity.

## Claims

1. A method of assaying an immune system response of a human or mammalian test subject that includes the following steps:

    (1) Preparing a peripheral blood lymphocyte population from a blood sample taken from said subject;

    (2) Exposing at least a portion of said population to predetermined amounts of amplifier TG or amplifier TGG and a mitogen or antigen;

    (3) Assaying the production in said population of lymphokine material induced by said mitogen or antigen, whereby an immune system assay result is provided that is representative of the amount of said subject's immune response to said mitogen or antigen.

2. The method of claim 1 wherein said subject is human.

3. The method of claim 2 wherein said means is PHA, pokeweed, Con A, or tetanus toxoid.

13

**4.** The method of claim 2 wherein said lymphokine material is IL-2 or gamma-interferon.

**5.** The method of claim 2 wherein said production is assayed by measuring thymidine incorporation.

**6.** The method of claim 5 wherein said thymidine is radioactively or fluorescently tagged.

**7.** The method of claim 1 wherein the procedure of said claim is performed a plurality of times with different portions of said sample, successively using different concentrations of said amplifier in step 2 of said claim, whereby a plurality of said immune system assay results are provided.

**8.** Use of the method according to claim 7 in a method for determining a dosage amount of amplifier TG or amplifier TGG that affects a cell-mediated immune system response, said method comprising
(1) Performing the method of claim 7 with a number of different concentrations of said amplifier to permit a determination to be made which of said immune system assay results represents the maximum immune response of which said subject's immune system is capable, whereby a maximum immune system assay result is provided;
(2) Comparing said subject's maximum immune system assay result with a reference immune system assay, said reference immune system assay being representative of a desired or predetermined level of immune response; and
(3) Determining the dosage amount of amplifier TG or amplifier TGG on the basis of the comparison made in step (2) of this claim.

**9.** Use of the method according to claim 8, wherein when said maximum immune system assay result is greater than said reference, the dosage amount of said amplifier is decreased.

**10.** Use of the method according to claim 8, wherein when said maximum immune system assay result is less than said reference, the dosage amount of said amplifier is increased but only within the nonparadoxical dosage range.

**11.** Use of the method according to claim 9 or 10, wherein said subject is human and suffers from an immunodeficient condition.

**12.** A method of titrating a dosage amount of amplifier TG or TGG to be administered to a person who has an immunodeficient condition, said method comprising the following steps:
(1) Preparing a peripheral blood lymphocyte population from a blood sample taken from said person;
(2) Performing the following steps a plurality of times, successively using different portions of said population:
(a) Exposing a said portion to a mitogen and amplifier TG or TGG, successively using different concentrations of said amplifier; and
(b) Assaying IL-2 production in said portion by measuring uptake of tagged thymidine, whereby a plurality of immune system assay results are provided in terms of a numerical quantity proportional to the respective amounts of said uptake;
(3) Comparing the maximum of said assay results with a predetermined reference immune system assay; and
(4) Titrating said dosage amount as follows:
(a) If said maximum assay result exceeds said reference assay, decreasing said dosage amount; and
(b) If said reference assay exceeds said maximum assay result, increasing said dosage amount but only within the nonparadoxical dosage range.

**13.** A method for determining, in regard to persons suffering from an immunodeficient condition associated with a systemic impairment of immune function, whether amplifier TG or TGG is to be administered to a said person, said method comprising the following steps:
(1) Preparing a peripheral blood lymphocyte population from a blood sample taken from said person;
(2) Performing the following steps a plurality of times, successively using different portions of said population:
(a) Exposing a said portion to a mitogen and amplifier TG or TGG, successively using different concentrations of said amplifier; and

14

(b) Assaying IL-2 production in said population by measuring uptake of tagged thymidine, whereby a plurality of immune system assay results are provided in terms of a numerical quantity proportional to the respective amounts of said uptake;

(3) Comparing the maximum of said assay results with a predetermined reference immune system assay, where said predetermined reference assay corresponds approximately to the immune function present in a person having 10% of normal T4 cell population, and where said reference assay is predetermined and adjusted upward or downward in accordance with conditions obtaining when the method is performed; and

(4) Making said determination as follows:

(a) If said maximum assay result exceeds said reference assay, amplifier TG or TGG is to be administered; and

(b) If said reference assay exceeds said maximum assay result, amplifier TG or TGG is not to be administered.

14. The method of claim 1, adapted to measure suppressor function, wherein:

(2) In step 2 of claim 1, said mitogen or antigen is a means for activating suppressor cells, and said step is performed a plurality of times successively using different portions of said population and successively using different concentrations of amplifier, whereby a plurality of suppressor-activated preparations is provided;

(2) The following steps 2-a through 2-e are inserted between steps 2 and 3 of claim 1, and they are carried out with regard to each said suppressor-activated preparation:

(a) Culturing said suppressor-activated preparation, thereby producing a suppressor-activated culture;

(b) Exposing said culture to a means for preventing cell division, and thereafter eliminating any residual amount of said means from said culture, thereby producing a nonproliferating culture;

(c) Mixing said nonproliferating culture with a peripheral blood lymphocyte population, thereby providing a responder preparation;

(d) Exposing a portion of said responder preparation to a means for inducing a cell proliferation immune response, and not exposing a further portion of said responder preparation to said means, thereby providing an exposed responder preparation and an unexposed responder preparation; and

(e) Culturing said exposed responder preparation and said unexposed responder preparation, thereby producing an exposed responder culture and an unexposed responder culture; and

(3) step 3 of claim 1 is an assay of cell proliferation in each said responder culture; and said index of immune system response is an index of said cell proliferation.

15. The method of claim 14 wherein:

only a portion of said peripheral blood lymphocyte population is exposed to said means for activating suppressor cells and a nonexposed portion is reserved; and said reserved portion is used as a peripheral blood lymphocyte population in step 2-c of said claim.

**Patentansprüche**

1. Verfahren zur Analyse einer Immunsystemantwort eines menschlichen oder Säugetier-Testobjekts, das folgende Schritte aufweist:

(1) Präparieren einer Peripherblut-Lymphocytenpopulation aus einer dem Objekt entnommenen Blutprobe;

(2) Aussetzen von wenigstens einem Teil dieser Population vorbestimmten Mengen von Verstärker TG oder Verstärker TGG und einem Mitogen oder Antigen;

(3) Analysieren der durch das Mitogen oder Antigen induzierten Produktion von Lymphokinmaterial in der Population, wodurch ein Immunsystemassayergebnis geliefert wird, das für die Stärke der Immunantwort des Objekts auf das Mitogen oder Antigen repräsentativ ist.

2. Verfahren nach Anspruch 1, wobei das Objekt ein Mensch ist.

3. Verfahren nach Anspruch 2, wobei das Mittel PHA, Kermesbeere, Con A oder Tetanustoxoid ist.

4. Verfahren nach Anspruch 2, wobei das Lymphokinmaterial IL-2 oder Gamma-Interferon ist.

15

**5.** Verfahren nach Anspruch 2, wobei die Produktion durch Messen von Thymidineinlagerung analysiert wird.

**6.** Verfahren nach Anspruch 5, wobei das Thymidin radioaktiv oder fluoreszent markiert wird.

**7.** Verfahren nach Anspruch 1, wobei der Vorgang des genannten Anspruchs eine Vielzahl von Malen mit verschiedenen Anteilen der Probe durchgeführt wird, wobei nacheinander verschiedene Konzentrationen des Verstärkers in Schritt 2 des Anspruchs eingesetzt werden, wodurch eine Vielzahl der Immunsystemassayergebnisse geliefert wird.

**8.** Anwendung des Verfahrens nach Anspruch 7 bei einem Verfahren zum Bestimmen einer Dosierungsmenge von Verstärker TG oder Verstärker TGG, die eine zellvermittelte Immunsystemantwort beeinflußt, wobei das Verfahren folgende Schritte aufweist:

(1) Durchführen des Verfahrens von Anspruch 7 mit einer Reihe von verschiedenen Konzentrationen des Verstärkers, um eine Bestimmung zu ermöglichen, welches der Immunsystemassayergebnisse die maximale Immunantwort repräsentiert, zu der das Immunsystem des Objekts fähig ist, wodurch ein maximales Immunsystemassayergebnis geliefert wird;

(2) Vergleichen des maximalen Immunsystemassayergebnisses des Objekts mit einem Referenz-Immunsystemassay, wobei der Referenz-Immunsystemassay einen gewünschten oder vorbestimmten Immunantwortpegel repräsentiert; und

(3) Bestimmen der Dosierungsmenge von Verstärker TG oder Verstärker TGG auf der Basis des in Schritt 2 dieses Anspruchs vorgenommenen Vergleichs.

**9.** Anwendung des Verfahrens nach Anspruch 8, wobei, wenn das maximale Immunsystemassayergebnis höher als der Referenzwert ist, die Dosierungsmenge des Verstärkers verringert wird.

**10.** Anwendung des Verfahrens nach Anspruch 8, wobei, wenn das maximale Immunsystemassayergebnis niedriger als der Referenzwert ist, die Dosierungsmenge des Verstärkers erhöht wird, jedoch nur innerhalb des nichtparadoxen Dosierungsbereichs.

**11.** Anwendung des Verfahrens nach Anspruch 9 oder 10, wobei das Objekt ein Mensch ist und an einem Immundefektzustand leidet.

**12.** Verfahren zum Titrieren einer Dosierungsmenge von Verstärker TG oder TGG, die einer Person mit einem Immundefektzustand zu verabreichen ist, wobei das Verfahren die folgenden Schritte aufweist:

(1) Präparieren einer Peripherblut-Lymphocytenpopulation aus einer der Person entnommenen Blutprobe;

(2) Durchführen der folgenden Schritte eine Vielzahl von Malen, wobei nacheinander verschiedene Anteile der Population eingesetzt werden:

(a) Aussetzen eines solchen Anteils einem Mitogen und Verstärker TG oder TGG, wobei nacheinander verschiedene Konzentrationen des Verstärkers eingesetzt werden; und

(b) Analysieren der IL-2-Produktion in dem Anteil durch Messen der Aufnahme von markiertem Thymidin, wodurch eine Vielzahl von Immunsystemassayergebnissen in Form einer Zahlengröße geliefert wird, die den jeweiligen Mengen der Aufnahme proportional ist;

(3) Vergleichen des Maximums der Assayergebnisse mit einem vorbestimmten Referenz-Immunsystemassay; und

(4) Titrieren der Dosierungsmenge wie folgt:

(a) wenn das maximale Assayergebnis den Referenzassay überschreitet, Verringern der genannten Dosierungsmenge; und

(b) wenn der Referenzassay das maximale Assayergebnis überschreitet, Erhöhen der genannten Dosierungsmenge, jedoch nur innerhalb des nichtparadoxen Dosierungsbereichs.

**13.** Verfahren zum Bestimmen in bezug auf Personen, die an einem Immundefektzustand leiden, der mit einer systemischen Immunfunktionsstörung verbunden ist, ob einer solchen Person Verstärker TG oder TGG zu verabreichen ist, wobei das Verfahren die folgenden Schritte aufweist:

(1) Präparieren einer Peripherblut-Lymphocytenpopulation aus einer der Person entnommenen Blutprobe;

(2) Durchführen der folgenden Schritte eine Vielzahl von Malen, wobei nacheinander verschiedene

16

Anteile der Population eingesetzt werden:

(a) Aussetzen eines solchen Anteils einem Mitogen und Verstärker TG oder TGG, wobei nacheinander verschiedene Konzentrationen des Verstärkers eingesetzt werden; und

(b) Analysieren der IL-2-Produktion in der Population durch Messen der Aufnahme von markiertem Thymidin, wodurch eine Vielzahl von Immunsystemassayergebnissen in Form einer Zahlengröße geliefert wird, die den jeweiligen Mengen der Aufnahme proportional ist;

(3) Vergleichen des Maximums der Assayergebnisse mit einem vorbestimmten Referenz-Immunsystemassay, wobei der vorbestimmte Referenzassay ungefähr der Immunfunktion entspricht, die in einer Person mit 10 % normaler T4-Zellenpopulation vorhanden ist, und wobei der Referenzassay vorbestimmt ist und nach Maßgabe von Bedingungen, die während der Durchführung des Verfahrens erhalten werden, nach oben oder unten eingestellt wird; und

(4) Durchführen der Bestimmung wie folgt:

(a) wenn das maximale Assayergebnis den Referenzassay überschreitet, ist Verstärker TG oder TGG zu verabreichen; und

(b) wenn der Referenzassay das maximale Assayergebnis überschreitet, ist kein Verstärker TG oder TGG zu verabreichen.

**14.** Verfahren nach Anspruch 1, das geeignet ist, die Suppressorfunktion zu messen, wobei:

(1) in Schritt 2 von Anspruch 1 das Mitogen oder Antigen ein Mittel zum Aktivieren von Suppressorzellen ist und der genannte Schritt eine Vielzahl von Malen durchgeführt wird, wobei nacheinander verschiedene Anteile der Population und nacheinander verschiedene Konzentrationen von Verstärker eingesetzt werden, wodurch eine Vielzahl von suppressoraktivierten Zubereitungen geliefert wird;

(2) die nachfolgenden Schritte 2-a bis 2-e zwischen die Schritte 2 und 3 von Anspruch 1 eingeschaltet und hinsichtlich jeder der genannten suppressoraktivierten Zubereitungen durchgeführt werden:

(a) Züchten der suppressoraktivierten Zubereitung, wodurch eine suppressoraktivierte Kultur erzeugt wird;

(b) Aussetzen der Kultur einem Mittel zum Verhindern von Zellteilung und anschließendes Beseitigen jeglicher Restmenge des genannten Mittels aus der Kultur, wodurch eine nichtproliferierende Kultur erzeugt wird;

(c) Mischen der nichtproliferierenden Kultur mit einer Peripherblut-Lymphocytenpopulation, wodurch eine Responderzubereitung gebildet wird;

(d) Aussetzen eines Anteils der Responderzubereitung einem Mittel zum Induzieren einer Zellproliferationsimmunantwort und Nichtaussetzen eines weiteren Anteils der Responderzubereitung dem genannten Mittel, wodurch eine ausgesetzte Responderzubereitung und eine nichtausgesetzte Responderzubereitung erhalten werden; und

(e) Züchten der ausgesetzten Responderzubereitung und der nichtausgesetzten Responderzubereitung, wodurch eine ausgesetzte Responderkultur und eine nichtausgesetzte Responderkultur erzeugt werden; und

(3) Schritt 3 von Anspruch 1 ein Assay von Zellproliferation in jeder der Responderkulturen ist; und der Index der Immunsystemantwort ein Index der Zellproliferation ist.

**15.** Verfahren nach Anspruch 14, wobei:

nur ein Teil der Peripherblut-Lymphocytenpopulation dem Mittel zum Aktivieren von Suppressorzellen ausgesetzt wird und ein nichtausgesetzter Teil zurückbehalten wird; und der zurückbehaltene Teil in Schritt 2-c des genannten Anspruchs als eine Peripherblut-Lymphocytenpopulation eingesetzt wird.

**Revendications**

**1.** Procédé pour essayer la réponse du système immunitaire d'un sujet de test, personne humaine ou mammifère, qui comporte les stades suivants :

(1) préparer une population de lymphocytes du sang périphérique à partir d'un échantillon de sang prélevé sur ledit sujet;

(2) exposer au moins une portion de ladite population à des quantités prédéterminées d'un amplificateur TG ou d'un amplificateur TGG et d'un mitogène ou d'un antigène;

(3) essayer la production dans ladite population de lymphokines induites par ledit mitogène ou antigène, grâce à quoi on obtient un résultat de l'essai du système immunitaire qui est représentatif du degré de la réponse immunitaire dudit sujet audit mitogène ou antigène.

**2.** Procédé selon la revendication 1, dans lequel ledit sujet est une personne humaine.

**3.** Procédé selon la revendication 2, dans lequel lesdits moyens sont PHA, le phytolaque à dix étamines, CONA ou la toxoïde du tétanos.

**4.** Procédé selon la revendication 2, dans lequel ladite lymphokine est IL-2 ou gamma interféron.

**5.** Procédé selon la revendication 2, dans lequel ladite production est essayée en mesurant l'incorporation de thymidine.

**6.** Procédé selon la revendication 5, dans lequel ladite thymidine est marquée par radio-activité ou par fluorescence.

**7.** Procédé selon la revendication 1, dans lequel la procédure de cette revendication est effectuée plusieurs fois avec différentes portions dudit échantillon, en utilisant successivement différentes concentrations dudit amplificateur du stade 2 de ladite revendication, d'où il résulte qu'on obtient plusieurs résultats d'essais du système immunitaire.

**8.** Utilisation du procédé selon la revendication 7, dans un procédé pour déterminer une quantité dosée d'amplificateur TG ou d'amplificateur TGG qui affecte une réponse du système immunitaire à médiation cellulaire, ledit procédé comprenant les stades suivants :

(1) mettre en oeuvre le procédé de la revendication 7 avec un certain nombre de concentrations différentes dudit amplificateur pour permettre d'effectuer une détermination qui, parmi les résultats d'essais du système immunitaire, représentent la réponse immunitaire maximale dont le système immunitaire du sujet est capable, grâce à quoi on obtient un résultat d'essai du système immunitaire maximal;

(2) comparer ledit résultat d'essai du système immunitaire maximal du sujet avec un essai de système immunitaire de référence, ledit essai du système immunitaire de référence étant représentatif d'un niveau désiré ou prédéterminé de réponse immunitaire; et

(3) déterminer la quantité dosée d'amplificateur TG ou d'amplificateur TGG sur la base de la comparaison effectuée au stade 2 de cette revendication.

**9.** Utilisation du procédé selon la revendication 8, dans laquelle, lorsque le résultat de l'essai du système immunitaire maximal est supérieur à ladite référence, on diminue la quantité dosée dudit amplificateur.

**10.** Utilisation du procédé selon la revendication 8, dans laquelle, lorsque le résultat de l'essai du système immunitaire maximal est inférieur à ladite référence, on augmente la quantité dosée dudit amplificateur, mais seulement à l'intérieur d'une plage de dosages non paradoxale.

**11.** Utilisation du procédé selon la revendication 9 ou la revendication 10, dans laquelle le sujet est une personne humaine et souffre d'un état immunodéficient.

**12.** Procédé pour titrer une quantité dosée d'amplificateur TG ou TGG à administrer à une personne qui a un état immunodéficient, ce procédé comprenant les stades suivants :

(1) préparer une population de lymphocytes du sang périphérique à partir d'un échantillon de sang prélevé sur cette personne;

(2) effectuer les stades suivants une multiplicité de fois, en utilisant successivement différentes portions de ladite population :

(a) exposer une portion d'un mitogène et d'un amplificateur TG ou TGG, en utilisant successivement différentes concentrations dudit amplificateur; et

(b) tester la production d'IL-2 dans ladite portion en mesurant la prise de thymidine marquée, grâce à quoi on obtient une multiplicité de résultats d'essais du système immunitaire en termes d'une quantité numérique proportionnelle aux quantités respectives de cette prise;

(3) comparer le maximum desdits résultats d'essais avec un essai du système immunitaire de référence prédéterminé; et

(4) titrer ladite quantité dosée comme suit :

(a) si ledit résultat d'essai maximal est supérieur à l'essai de référence, diminuer ladite quantité dosée; et

(b) si ledit essai de référence est supérieur au résultat d'essai maximal, augmenter la quantité dosée, mais seulement à l'intérieur d'une plage de dosages non paradoxale.

13. Procédé pour déterminer, en ce qui concerne des personnes souffrant d'un état immunodéficient associé à une détérioration systémique de la fonction immunitaire, si l'amplificateur TG ou TGG doit être administré à une telle personne, ce procédé comprenant les stades suivants :

(1) préparer une population de lymphocytes du sang périphérique à partir d'un échantillon de sang prélevé sur ladite personne;

(2) effectuer les stades suivants une multiplicité de fois, en utilisant successivement différentes portions de ladite population :

(a) exposer une de ces portions à un mitogène et à un amplificateur TG ou TGG, en utilisant successivement différentes concentrations dudit amplificateur; et

(b) tester la production d'IL-2 dans ladite population en mesurant la prise de thymidine marquée, grâce à quoi on obtient une multiplicité de résultats d'essais du système immunitaire en termes d'une quantité numérique proportionnelle aux quantités respectives de ladite prise;

(3) comparer le maximum des résultats d'essais avec un essai du système immunitaire de référence prédéterminé, ledit essai de référence prédéterminé correspondant approximativement à la fonction immunitaire présente chez une personne ayant 10% de la population normale de cellules T4, et cet essai de référence étant prédéterminé et ajusté vers le haut ou vers le bas en fonction des conditions obtenues lorsqu'on met en oeuvre le procédé; et

(4) effectuer cette détermination comme suit :

(a) si le résultat d'essai maximal est supérieur à l'essai de référence, l'amplificateur TG ou TGG doit être administré; et

(b) si cet essai de référence est supérieur au résultat d'essai maximal, l'amplificateur TG ou TGG ne doit pas être administré.

14. Procédé selon la revendication 1, adapté pour mesurer une fonction de suppresseur, dans lequel :

(1) dans le stade 2 de la revendication 1, le mitogène ou l'antigène est un moyen pour activer des cellules suppresseurs et ce stade est mis en oeuvre une multiplicité de fois en utilisant successivement différentes portions de ladite population et en utilisant successivement des concentrations différentes d'amplificateur, d'où il résulte qu'on obtient une multiplicité de préparations de suppresseurs activés;

(2) les stades suivants 2-a à 2-e sont introduits entre les stades 2 et 3 de la revendication 1, et sont ensuite mis en oeuvre en ce qui concerne chaque préparation de suppresseur activé :

(a) cultiver ladite préparation de suppresseur activé, produisant ainsi une culture de suppresseur activé;

(b) exposer ladite culture à un moyen pour empêcher la division des cellules et éliminer ensuite de ladite culture toute quantité résiduelle de ces moyens, produisant ainsi une culture non proliférante;

(c) mélanger ladite culture non proliférante à une population de lymphocytes du sang périphérique, procurant ainsi une préparation de répondant;

(d) exposer une portion de ladite préparation de répondant à un moyen pour induire une réponse immunitaire à la prolifération de cellules, et ne pas exposer une autre portion de ladite préparation de répondant à ce moyen, procurant ainsi une préparation de répondant exposée et une préparation de répondant non exposée; et

(e) cultiver ladite préparation de répondant exposée et ladite préparation de répondant non exposée, produisant ainsi une culture de répondant exposée et une culture de répondant non exposée; et

3 le stade 3 de la revendication 1 est un test de la prolifération de cellules dans chacune des cultures de répondant; et l'indice de réponse du système immunitaire est un indice de cette prolifération de cellules.

15. Procédé selon la revendication 14, dans lequel seule une portion de la population de lymphocytes du sang périphérique est exposée aux moyens pour activer les cellules suppresseurs et dans lequel on réserve une portion non exposée et dans lequel ladite portion réservée est utilisée comme population de lymphocytes du sang périphérique dans le stade 2-c de ladite revendication.